# EUROPEAN PATENT SPECIFICATION

(11) **EP 1 145 010 B1**
(45) Date of publication and mention of the grant of the patent: **15.06.2005**
(21) Application number: 00901629.6
(22) Date of filing: 18.01.2000
(51) Int. Cl.: G01N 33/543

(54) **PURIFICATION PROCESS USING MAGNETIC PARTICLES**
REINIGUNGSVERFAHREN UNTER VERWENDUNG VON MAGNETISCHEN PARTIKELN
TECHNIQUE DE PURIFICATION FAISANT INTERVENIR DES PARTICULES MAGNETIQUES

(30) Priority: 18.01.1999 FI 990082
(43) Date of publication of application: 17.10.2001
(73) Proprietor: Thermo Electron Oy, 01620 Vantaa (FI)
(72) Inventor: SEPPÄNEN, Helena, FIN-00950 Helsinki (FI); PALOMÄKI, Pekka, FIN-02360 Espoo (FI); TUUNANEN, Jukka, FIN-00630 Helsinki (FI); KÄRMENIEMI, Timo, FIN-00820 Helsinki (FI)
(74) Representative: Heikkinen, Esko Juhani
(86) International application number: PCT/FI2000/000031
(87) International publication number: WO 2000/042432

(56) References cited:
- WO-A1-96/12959
- WO-A1-98/31840
- WO-A2-96/18731
- US-A- 5 705 628
- ANIL WIPAT ET AL.: 'Monoclonal antibodies for Streptomyces lividans and their use for immunomagnetic capture of spores from soil' MICROBIOLOGY vol. 140, 1994, pages 2067 - 2076, XP002927468

## Description

### Background of the invention

The present invention relates to the purification of biological substances using magnetic particles which bind the material specifically from a mixture. The invention can be used for instance for purifying nucleic acids DNA or RNA.

Magnetic particles can be coated with a separation reagent which reacts specifically with a desired biological substance. The particles and the bound substance are separated from the mixture and thereafter the substance is released from the particles for further prosecution. Nowadays this is done in practice so that the particles are drawn with a magnet against the wall of the vessel containing the mixture, and the liquid is poured or sucked off the vessel. Thereafter a new liquid can be dispensed into the vessel. Manual or automatic equipments for such separation technology are also commercially available (e.g., Spherotec, Inc., AutoMag Processor (USA), Merck Magnetic Rack (Darmstadt, Germany), PerSeptive Biosystems 96 well plate separator, Multi-6 Separator, Solo-Sep Separator (USA), Dynal Magnetic Particles Concentrators).

The old purification technique for DNA involves ultracentrifugation in a dense cesium chloride gradient. However, also magnetic particle technology described above has been used for purifying nucleic acids.

WO 94/18565 (Labsystems Oy) suggests a method and device for magnetic particle specific binding assay, in which magnetic particles are separated from a mixture by a probe comprising a rod movable in a vertical bore and provided with a magnet at the lower end thereof. The probe is pushed into the mixture with the rod in the lower position, whereby the particles are collected on the probe. Then the probe is transferred to another vessel and the rod is pulled in its upper position, whereby the particles are released. Thus all steps of the assay can be carried out in a separate vessel without having to transfer liquids. In the last vessel, a measurement is carried out.

WO 96/12959 (Labsystems Oy) suggests a magnetic particle transfer tool comprising an elongated body with a concavely tapered tip part. The body further comprises means for providing a longitudinal magnetic field to collect particles to the tip of the body. The magnetic field can be eliminated in order to release the particles. This tool can be used especially for collecting particles from a large volume and releasing them into a very small volume.

### General description of the invention

Now a method according to claim 1 has been invented. Some preferable embodiments of the invention are defined in the other claims.

According to the invention, material to be purified is dispensed in a first medium containing magnetic particles, which have been coated with a binding reagent for the material. The binding reaction takes place, after which the particles are separated by means of a magnetic probe and transferred into a second medium, in which a desired further reaction necessary for the purification may take place. The particles can be transferred similarly via further mediums for carrying out further steps of the purification process. All the vessels may contain the necessary reaction medium ready when the particles are transferred into it. Preferably the particles are also released from the probe in the second and subsequent mediums.

According to the invention, at least one of the mediums contains a surface tension releasing agent. This promotes the complete collection of the particles.

Some of such agents have been used also before in this connection to promote the binding of the substance to be separated, see e.g. Wipat et al., Microbiology (1994), 140, 2067. In these known methods, the particles are not transferred from a vessel to another bu they are held on the wall of the vessel by means of a external magnet, while the medium is removed from the vessel.

The invention can be used especially for purifying nucleic acids, such as ssDNA, dsDNA, and mRNA.

### Brief description of the drawings

The enclosed drawings form a part of the written description.
Figure 1 shows the effect of a detergent in collecting and releasing steps of magnetizable particles.
Figure 2 shows the effect of salt and saccharose in collecting and releasing buffer.
Figure 3 shows the effect of protein in collecting and releasing buffer.
Figure 4 shows the effect of a detergent when magnetic particles of different suppliers were used.

### Detailed description of the invention

The invention can be used for instance for the purification of cells, viruses, subcellular organelles, proteins, and especially nucleic acid materials.

The magnetic particles are preferably paramagnetic. The size of the particles is usually less than 50 µm, preferably 0.1 - 10 µm, and most preferably 1 - 5 µm. The concentration of the particles may be eg. 0.01 - 5 mg/ml, preferably 0.05 - 3 mg/ml, and most preferably 0.2 - 2 mg/ml.

The particles have been coated or treated with a binding reagent, eg. silicon, lectins and/or other reactive functional groups such a oligonucleotides, antibodies, antigens, streptavidin, or biotin.

The particles are preferably transferred from a vessel to another by using a probe comprising a rod movable in a vertical bore and provided with a magnet at the lower end thereof. The probe is pushed into the mixture with the rod in the lower position, whereby the particles are collected on the probe. Then the probe is transferred to another vessel, and when the rod is pulled to its upper position the particles are released.

Different kind of surface tension releasing compounds, especially water soluble compounds, can be used in the method. Examples of such are:
A. Tensides, such as
   - Soaps
   - Detergents; including anionic, kationic, non-ionic and zwitterionic compounds
B. Alcohols, such as
   - Polyethylene and polyvinyl alcohols and their protein etc. derivatives
C. Proteins
D. Salts and carbohydrates in high concentrations, such as
   - NaCl
   - Saccharose
Also mixtures of compounds can be used.

Especially tensides such as detergents are suitable. Preferable detergents are ethoxylated anhydrosorbitol esters. The esters may contain eg. about 4 - 20 ethylene oxide groups.

The concentration of a tenside may be eg. 0.001 - 0.5% (w/v), preferably 0.005 - 0.1% (w/v), and most preferably 0.01 - 0.05% (w/v). The concentration of a protein may be eg. 0.1 - 10% (w/v), preferably 0.25 - 5% (w/v), and most preferably 0.5 - 2% (w/v). The concentraion of a salt may be eg. 0.1 - 10 M, preferably 0.1 - 7 M.

For purification of DNA or mRNA from different sources (for instance, DNA from PCR amplification; DNA from blood, bone marrow or cultured cells; mRNA from eucaryotic total RNA or from crude extracts of animal tissues, cells and plants) the nucleic acids are immobilized by using magnetic particles. The binding can be mediated by the interaction of streptavidin and biotin, whereby particles coated with streptavidin and biotinylated DNA can be used. In addition, DNA can be adsorbed to the surface of the particles. The binding of mRNA can be mediated by Oligo (dT)₂₅ covalently coupled to the surface of the particles.

After the immobilization, the nucleic acids are washed several times to remove all the reaction components resulting from the amplification or other contaminants and, e.g., PCR inhibitors.

The washing can be performed by releasing and collecting complexes in a washing buffer and by transfering the complexes to another well containing fresh washing buffer.

For ssDNA purification the immobilized double-stranded DNA can be converted to a single-stranded by incubation with 0.1 M NaOH and using magnetic separation.

For the isolation of mRNA, it can be eluted from the particles by using a low salt buffer.

The purification process can be performed by a magnetic particle processor, in which all the mediums are ready in separate vessel. A surface tension releasing compound is preferably used in each medium. Suitable disposable plates, such as microtitration plates, comprising the necesary vessels can be used. In one plate, several parallel purifications can be accomplished.

### Example of reagents used for a ssDNA purification

1. Particle suspension in eg. phosphate, Tris or Borate buffered saline, pH 7.4, containing 0.1% BSA, 15 mM NaN₃ and eg. 0.02% polyoxyethylene (20) sorbitan monolaurate (Tween 20 ™) as a surface tension releasing agent
2. Binding and Washing buffer (TEN): 10 mM Tris-HCl, 1 mM EDTA,
   2 M NaCl, eg. 0.02% Tween 20 ™, 15 mM NaN₃, pH 7.5
3. TE buffer: 10 mM Tris-HCl, 1 mM EDTA, eg. 0.02% Tween 20 ™, 15 mM NaN₃, pH 7.5
4. Melting solution: 0.1 M NaOH, eg. 0.02% Tween 20 ™
5. eg. 0.02% Tween 20 ™ in distilled water, 15 mM NaN₃

### Example of reagents used for a mRNA direct purification

1. Oligo (dT)₂₅ particle suspension in PBS, pH 7.4, containing eg. 0.02% Tween 20 ™ and 15 mM NaN₃
2. Lysis/binding buffer: 100 mM Tris-HCl, pH 8.0, 500 mM LiCl, 10 mM EDTA, 1% LiDS, 5 mM dithiothreitol (DTT), 15 mM NaN₃, (eg. 0.02% Tween 20 ™)
3. Washing buffer with LiDS (SDS): 10 mM Tris-HCl, pH 8.0, 0.15 M LiCl,
   1 mM EDTA, 0.1% LiDS, 15 mM NaN₃ (eg. 0.02% Tween 20 ™)
4. Washing buffer: 10 mM Tris-HCl, pH 8.0, 0.15 M LiCl, 1 mM EDTA,
   eg. 0.02% Tween 20 ™, 15 mM NaN₃
5. Elution solution: 2 mM EDTA, pH 8.0 , 15 mM NaN₃, eg. 0.02% Tween 20 ™
6. Reconditioning solution: 0.1 M NaOH , eg. 0.02% Tween 20 ™
7. Storage buffer Oligo (dT)₂₅: 250 mM Tris-Hcl, pH 8, 20 mM EDTA, 0.1% Tween-20, 15 mM NaN₃

### Example of the reagents used for a mRNA purification

1. Binding buffer: 20 mM Tris-HCl, pH 7.5, 1.0 M LiCl, 2 mM EDTA, 15 mM NaN₃ , eg. 0.02% Tween 20 ™
2. Washing buffer: 10 mM Tris-HCl, pH 8.0, 0.15 mM LiCl, 1 mM EDTA, 15 mM NaN₃, eg. 0.02% Tween 20 ™
5. Elution solution: 2 mM EDTA, pH 8.0 , 15 mM NaN₃, eg. 0.02% Tween 20 ™

### Example of reagents used for RNA isolation

1. 4 M guanidium isothiocyanate, 25 mM sodium citrate pH 7.0, 0.5% N-lauryl sarcosine, 0.01 M β-mercaptoethanol

### Example of reagents used for a DNA direct purification

1. Particle suspension in Lysis buffer (eg. 50 mM Tris-HCl, pH 7.2, 50 mM EDTA, 3% SDS, 1% 2-mercaptoethanol; 50 mM KCl, 10 - 20 mM Tris-HCl, 2.5 mM MgCl₂, pH 8.3, 0.5 Tween 20 ™, 100 µg/ml Proteinase K; 100 mM Tris-HCl, pH 8.5, 5 mM EDTA, 1% SDS, 500 µg/ml Proteinase K) containing 15 mM NaN₃
2. Washing buffer containing 15 mM NaN₃ and eg. 0.02% Tween 20 ™
3. Resuspension buffer containing 15 mM NaN₃ and eg. 0.02% Tween 20 ™

### Example of the purification process of PCR products by a magnetic particle processor at room temperature

The reagents are dispensed into a subsequent wells of a plate.

### Example of a reagent configuration:

- Well 1.: Sample (biotinylated DNA, PCR amplicons)
- Well 2.: Streptavidin coated magnetic particles in washing buffer
- Wells 3 - 5.: Washing buffer
- Well 6.: NaOH
- Well 7.: TE buffer
- Well 8.: Distilled water

### Example of processing steps:

Well 2. Mixing, washing and collecting of particles, moving of them into well 3
Well 3. Washing of particles, moving of them into well 4
Well 4. Washing of particles, moving of them into well 1
Well 1. Sample incubation 10', moving of particles into well 4
Well 4. Washing of particles, moving of them into well 5
Well 5. Washing of particles, moving of them into well 6
Well 6. Incubation 5' in melting solution, moving of particles into well 4
Well 4. Washing of particles, moving of them into well 5
Well 5. Washing of particles, moving of them into well 7
Well 7. Rinsing of particles, moving of them into well 8
Well 8. Releasing of particles

### The effect of surface tension releasing agent (STRA) in collecting and releasing steps of magnetizable particles

Streptavidin coated magnetic particles (sizes: Scigen streptavidin 3 µm; Scigen; SPHERO™ streptavidin 4 - 4.5µm, Spherotec, Dynabeads M-280 streptavidin 2.8 µm, Dynal) were saturated with biotinylated alkaline phosphatase (Calbiochem, San Diego, CA) for 1 h at +37 °C. Saturated particles were first washed to remove the unbound alkaline phosphatase and were then used to examine the effect of STRA in collecting and releasing steps of a magnetic particle processor. The instrument settings of these examples were adjusted from 20 µl to 200 µl and the capacity range of the processor was 1 - 24 samples per run. The processor utilized a rod magnet (cylindrical NdFeB axially magnetized, length 2 mm, width 3 mm) in polypropene tube (outer width 4.5 mm).

Briefly, the particles were processed by releasing and collecting them from well to well so that the whole process comprised of 10 steps. The amount of particles, which remained into the wells after the collection, was estimated with alkaline phosphatase assay. Samples (10 µl) from each well were transferred to an empty microtitration plate (round-bottomed wells, width 6.5 mm).

In this assay alkaline phosphatase saturated particles (0.016 µg - 1 µg particles / 10 µl diluent) were used as standards. Into the wells containing 10 µl samples and standards were added 100 µl pNPP-substrate diluted in diethanolamine (DEA) buffer (Labsystems). The substrate was incubated for 15 minutes at +37 °C with continuous shaking (900 rpm) in Labsystems iEMS Incubator/Shaker. The reaction was stopped by adding 100 µl 1M NaOH into each well and the absorbances at 405 nm were measured by photometer (Labsystems Multiskan).

The amount of remaining particles was determined from a linear standard curve and finally results were expressed as percentage of initial amount of particles (0.2 mg/well).

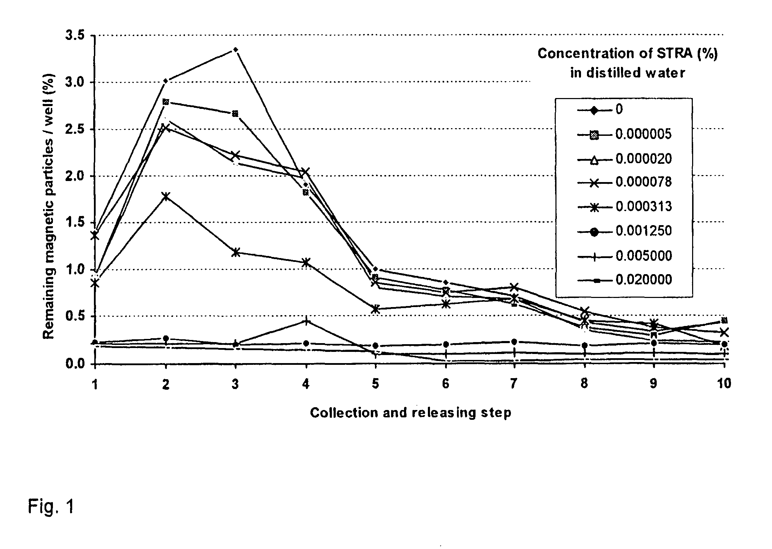

In Fig 1. is shown the effect of detergent (Tween 20 ™) in different concentrations. The degree of remaining particles (Scigen streptavidin) were over 3% / well, when surface tension releasing agent was not added into the collecting and releasing buffer. When the detergent concentration was ≥ 0.00125%, the particles were collected efficiently.

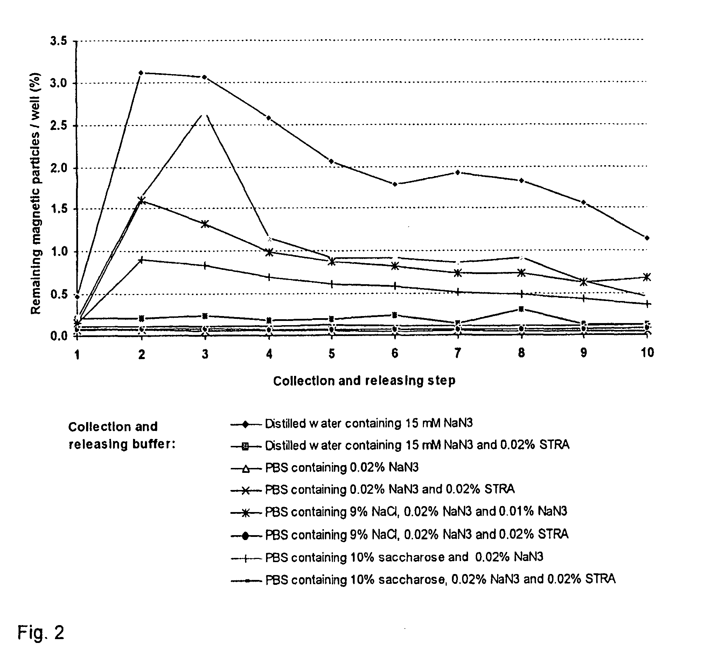

In Fig 2. is shown the effect of salt and saccharose in collecting and releasing buffer. By adding these components into the buffer, the collection of particles (Scigen streptavidin) was more efficient.

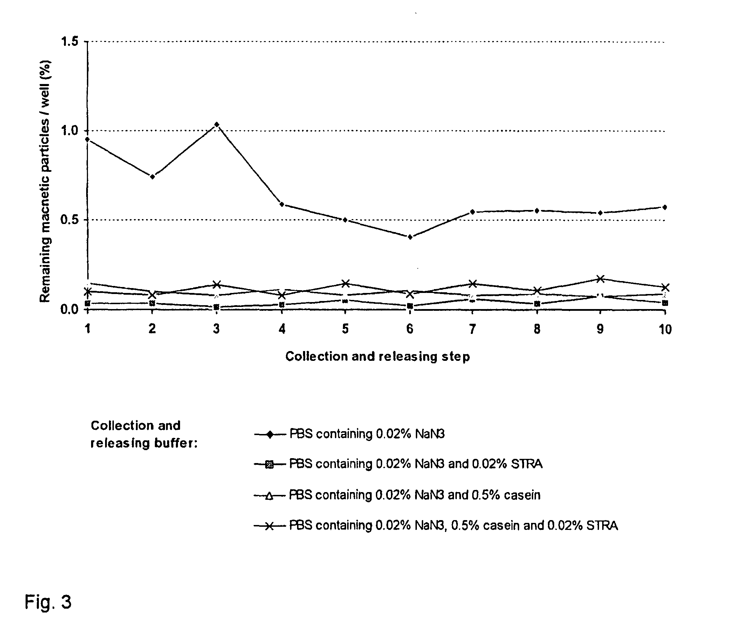

In Fig 3. is shown the effect of a protein (casein) which was improving the collecting steps of particles (SPHERO™ streptavidin) in some degree.

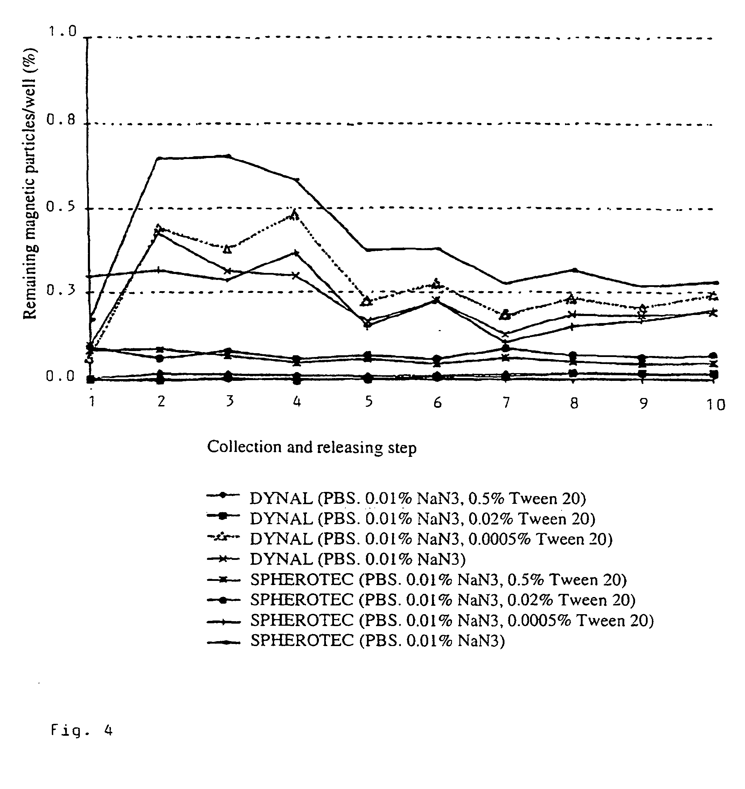

In Fig. 4 is shown the effect of detergent (Tween 20 ™) when the magnetic particles of different suppliers were used.

## Claims

1. A method for the purification of a substance, wherein
- material containing the substance, and magnetic particles coated or treated with a reagent which binds the particles to the substance are dispensed in a first liquid medium,
- a binding reaction is let to take place, in which reaction the substance is bound to the particles, and
- a magnetic probe is pushed into the medium, whereby the particles adhere to the probe, and the probe together with the particles and the substance bound to them is transferred to a second liquid medium, and if desired, separated from the second medium and transferred to a third liquid medium,
**characterized in that**
- for improving the adherence of the particles from the mediums to the probe, a surface tension releasing agent is dispensed at least to one of the mediums, preferably at least to the first medium, and most preferably to all mediums, before the probe and the particles are transferred from it.

2. A method according to claim 1, wherein the surface releasing compound is a tenside, alcohol, protein, or a salt or carbohydrate.

3. A method according to claim 1 or 2, wherein the surface tension releasing compound is a tenside, such as a detergent.

4. A method according to claim 3, wherein the concentration of the tenside is 0.001 - 0.5% (w/v), preferably 0.005 - 0.1% (w/v), and most preferably 0.01 - 0.05% (w/v).

5. A method according to claim 1 or 2, wherein the surface tension releasing compound is a protein.

6. A method according to claim 5, wherein the concentration of the protein is 0.1 - 10% (w/v), preferably 0.25 - 5% (w/v), and most preferably 0.5 - 2% (w/v).

7. A method according to claim 1 or 2, wherein the surface tension releasing compound is a salt.

8. A method according to claim 7, wherein the concentration of the salt is 0.1 - 10 M, preferably 0.1 - 7 M.

9. A method according to any of claims 1-8 for the purification of cells, viruses, subcellular organelles, proteins, or nucleic acid materials.

10. A method according to claim 9 for the purification of nucleic acid materials.

11. A method according to any of claims 1-10, wherein the size of the magnetic particles is less than 50 µm, preferably 0.1 - 10 µm, and most preferably 1 - 5 µm.

12. A method according to any of claims 1-11, wherein the concentration of the magnetic particles is 0.01 - 5 mg/ml, preferably 0.05 - 3 mg/ml, and most preferably 0.2 - 2 mg/ml.

13. A method for separating magnetic particles by means of a magnetic probe from a liquid medium, **characterized in that**, for improving the adherence of the particles from the medium to the probe, a surface tension releasing agent is dispensed into the medium before the particles are separated.

14. A method for improving the adherence of magnetic particles from a liquid medium to a magnetic probe to be pushed into the medium, **characterized in that** a surface tension releasing agent is dispensed into the medium before the particles are adhered to the probe.

## Patentansprüche

1. Verfahren zum Reinigen einer Substanz, wobei
- Material, das die Substanz enthält, und magnetische Partikel, die mit einem Reagenz beschichtet oder behandelt sind, das die Partikel an die Substanz bindet, in einem ersten flüssigen Medium dispergiert werden,
- das Stattfinden einer Bindungsreaktion zugelassen wird, bei welcher Reaktion die Substanz an die Partikel gebunden wird, und
- eine magnetische Sonde in das Medium geschoben wird, wodurch die Partikel, die an der Sonde anhaften, und die Sonde zusammen mit den Partikeln und der Substanz, die daran angebunden ist, zu einem zweiten flüssigen Medium übertragen werden, und, falls es erwünscht ist, von dem zweiten Medium separiert und zu einem dritten flüssigen Medium übertragen werden,
**dadurch gekennzeichnet, dass**
- zum Verbessern des Anhaftens der Partikel von den Medien an der Sonde ein Oberflächenspannungslösungsmittel wenigstens in einem der Medien dispergiert wird, vorzugsweise wenigstens in dem ersten Medium, und am bevorzugtesten in allen Medien, bevor die Sonde und die Partikel davon übertragen werden.

2. Verfahren nach Anspruch 1, wobei die Oberflächenlösungszusammensetzung ein Tensid, Alkohol, Protein oder ein Salz oder Carbohydrat ist.

3. Verfahren nach Anspruch 1 oder 2, wobei die Oberflächenspannungslösungszusammensetzung ein Tensid, wie ein Detergent ist.

4. Verfahren nach Anspruch 3, wobei die Konzentration des Tensides 0,001 - 0,5 % (Gewicht/Volumen), vorzugsweise 0,005 - 0,1 % (Gewicht/Volumen) und am bevorzugtesten 0,01 - 0,05 % (Gewicht/Volumen) ist.

5. Verfahren nach Anspruch 1 oder 2, wobei die Oberflächenspannungslösezusammensetzung ein Protein ist.

6. Verfahren nach Anspruch 5, wobei die Konzentration des Proteins 0,1 - 10 % (Gewicht/Volumen), vorzugsweise 0,25 - 5 % (Gewicht/Volumen) und am bevorzugtesten 0,5 - 2 % (Gewicht/Volumen) ist.

7. Verfahren nach Anspruch 1 oder 2, wobei die Oberflächenspannungslösezusammensetzung ein Salz ist.

8. Verfahren nach Anspruch 7, wobei die Konzentration des Salzes 0,1 - 10 M, vorzugsweise 0,1 - 7 M ist.

9. Verfahren nach einem der Ansprüche 1 - 8 zum Reinigen von Zellen, Viren, subzellularen Organellen, Proteinen oder Nukleinsäurematerialien.

10. Verfahren nach Anspruch 9 zum Reinigen von Nukleinsäurematerialien.

11. Verfahren nach einem der Ansprüche 1 - 10, wobei die Größe der magnetischen Partikel geringer als 50 µm, vorzugsweise 0,1 - 10 µm, und am bevorzugtestens 1 - 5 µm ist.

12. Verfahren nach einem der Ansprüche 1 - 11, wobei die Konzentration der magnetischen Partikel 0,01 - 5 mg/ml, vorzugsweise 0,05 - 3 mg/ml, und am bevorzugtesten 0,2 - 2 mg/ml ist.

13. Verfahren zum Separieren von magnetischen Partikeln mittels einer magnetischen Sonde aus einem flüssigen Medium, **dadurch gekennzeichnet, dass** zum Verbessern des Anhaftens der Partikel von dem Medium an der Sonde ein Oberflächenspannungslösungsmittel in das Medium dispergiert wird, bevor die Partikel separiert werden.

14. Verfahren zum Verbessern des Anhaftens von magnetischen Partikeln von einem flüssigen Medium an einer magnetischen Sonde, die in das Medium zu drücken ist, **dadurch gekennzeichnet, dass** ein Oberflächenspannungslösungsmittel in das Medium dispergiert wird, bevor die Partikel an der Sonde anhaften.

## Revendications

1. Procédé de purification d'une substance, dans lequel
- un matériel contenant la substance, et des particules magnétiques revêtues ou traitées à l'aide d'un réactif qui lie les particules à la substance, sont introduits dans un premier milieu liquide,
- une réaction de liaison est amenée à avoir lieu, réaction dans laquelle la substance est liée aux particules, et
- une sonde magnétique est introduite dans le milieu, de sorte que les particules adhèrent à la sonde, et la sonde ensemble avec les particules et la substance liée à celles-ci est transférée dans un deuxième milieu liquide, et si on le souhaite, séparée du deuxième milieu et transférée dans un troisième milieu liquide,
**caractérisé en ce que**
- pour améliorer l'adhérence des particules depuis les milieux à la sonde, un agent de relâchement de tension superficielle est introduit dans au moins un des milieux, de préférence au moins le premier milieu, et de manière la plus préférée dans tous les milieux, avant que la sonde et les particules ne soient transférées depuis celui-ci.

2. Procédé selon la revendication 1, dans lequel le composé de relâchement de tension superficielle est un tensioactif, un alcool, une protéine, ou un sel ou hydrate de carbone.

3. Procédé selon la revendication 1 ou 2, dans lequel le composé de relâchement de tension superficielle est un tensioactif, tel qu'un détergent.

4. Procédé selon la revendication 3, dans lequel la concentration du tensioactif est de 0,001 à 0,5 % (p/v), de préférence de 0,005 à 0,1 % (p/v), et de manière la plus préférée de 0,01 à 0,05 % (p/v).

5. Procédé selon la revendication 1 ou 2, dans lequel le composé de relâchement de tension superficielle est une protéine.

6. Procédé selon la revendication 5, dans lequel la concentration de la protéine est de 0,1 à 10 % (p/v), de préférence de 0,25 à 5 % (p/v), et de manière la plus préférée de 0,5 à 2 % (p/v).

7. Procédé selon la revendication 1 ou 2, dans lequel le composé de relâchement de tension superficielle est un sel.

8. Procédé selon la revendication 7, dans lequel la concentration du sel est de 0,1 à 10 M, de préférence 0,1 à 7 M.

9. Procédé selon l'une quelconque des revendications 1 à 8 pour la purification de cellules, virus, organites sous-cellulaires, protéines, ou matériels d'acide nucléique.

10. Procédé selon la revendication 9, pour la purification de matériels d'acide nucléique.

11. Procédé selon l'une quelconque des revendications 1 à 10, dans lequel la taille des particules magnétiques est inférieure à 50 µm, de préférence de 0,1 à 10 µm, et de manière la plus préférée de 1 à 5 µm.

12. Procédé selon l'une quelconque des revendications 1 à 11, dans lequel la concentration des particules magnétiques est de 0,01 à 5 mg/ml, de préférence de 0,05 à 3 mg/ml, et de manière la plus préférée de 0,02 à 2 mg/ml.

13. Procédé pour séparer des particules magnétiques par l'intermédiaire d'une sonde magnétique depuis un milieu liquide, **caractérisé en ce que**, afin d'améliorer l'adhérence des particules depuis le milieu à la sonde, un agent de relâchement de tension superficielle est introduit dans le milieu avant que les particules ne soient séparées.

14. Procédé pour améliorer l'adhérence de particules magnétiques depuis un milieu liquide à une sonde magnétique à introduire dans le milieu, **caractérisé en ce qu'**un agent de relâchement de tension superficielle est introduit dans le milieu avant que les particules n'adhèrent à la sonde.
